# EUROPEAN PATENT SPECIFICATION

(11) **EP 3 634 502 B1**
(45) Date of publication and mention of the grant of the patent: **06.09.2023**
(21) Application number: 18732926.3
(22) Date of filing: 04.06.2018
(51) Int. Cl.: A61L 2/07, A61L 2/24, A61L 2/26

(54) **STERILIZING MACHINE FOR LOOSE PRODUCTS**
STERILISIERMASCHINE FÜR LOSE PRODUKTE
MACHINE DE STÉRILISATION POUR PRODUITS EN VRAC

(30) Priority: 05.06.2017 IT 201700061246
(43) Date of publication of application: 15.04.2020
(73) Proprietor: Icos Pharma S.p.A., 33080 Zoppola (IT)
(72) Inventor: BENI, Stefano, 33170 Pordenone (IT); CANNAS, Emiliano Maurizio, 26837 Mulazzano (IT)
(74) Representative: Petraz, Gilberto Luigi
(86) International application number: PCT/IT2018/050099
(87) International publication number: WO 2018/225106

(56) References cited:
- EP-A1- 3 064 226
- EP-A2- 1 920 853
- WO-A1-2007/014312
- WO-A1-2014/037831
- WO-A1-2015/150581
- DE-A1- 3 841 930
- JP-A- H11 290 061
- US-A- 5 397 535

## Description

### FIELD OF THE INVENTION

The present invention concerns a sterilizing machine for loose products, for example a sterilizing machine with a rotating drum which can be used, in particular, in the hospital, pharmaceutical or food field, or in the industrial sector in the production of the loose products.

The loose products can be, for example, closing stoppers for test tubes for laboratory analysis, closing stoppers for vials of drugs, or small instruments or objects used in operating theaters or laboratories, or other.

### BACKGROUND OF THE INVENTION

It is known that in the hospital or pharmaceutical field, or other fields, such as the food industry, it is necessary to use sterile products to prevent contamination or alteration of substances with which they come into contact, or to avoid introducing bacteria, germs, other corpuscles or potentially pathogenic or harmful agents into environments where their total absence is required.

Among such sterile products, in all the above mentioned fields, loose products, even small ones, are normally present, such as, for example, stoppers for closing test tubes intended to contain substances to be subjected to laboratory analysis, or small instruments or objects used in operating theaters or laboratories.

The loose products can already be sterile when they are produced and sold in packages which are themselves sterile, or are sterilized before each use and reused several times during their working life.

In some cases the loose products therefore require to be sterilized. Some examples of machines or devices for sterilizing objects are described for example in documents DE-A-3841930 e WO-A-2015150581.

In other documents, for example documents US-A-5397535 and WO-A-2007014312 operations to sterilize or disinfect objects are described in various ways, also used in the healthcare sector or suchlike.

Moreover, in document EP-A-1920853 a machine for cleaning and drying objects is described, while in document JP-A-H11290061 a heat treatment process of a pneumatic transport pipe is described.

Moreover, document EP-A-3064226 describes a known machine and method for washing and/or sterilizing loose products.

It is also known to sterilize these loose products in sterilizing machines provided with a sealed chamber in which a rotating drum receives the products to be sterilized from a so-called "dirty" loading side, it sterilizes them during a treatment cycle normally comprised between 1 and 5 hours, and unloads them from a so-called "sterile" unloading side, where they are generally packaged in sterile packs.

Rotating drums are also known, partitioned into sectors, each containing a desired quantity of loose products to be poured into the individual packs after sterilization.

As is also known, the process of treating loose products, even during unloading from the machine and the subsequent use of the loose products, must always guarantee an optimum condition of sterility, that is, it must prevent the treated loose products from being contaminated in any way, above all in the delicate step when they are unloaded from the sterilizing machine.

Normally, the treated loose products are taken from an unloading mouth of the treatment chamber and introduced, typically by operators appointed for this purpose, into bags or suchlike, which are then closed and taken to a subsequent station of use, for example a filling machine. This occurs both for sterilizing machines with a rotating drum and also for other types of sterilizing machines.

The loose products, during the delicate unloading step, can therefore be subject to unwanted contamination, therefore the sterilization conditions required are inevitably and dangerously interrupted.

Another problem with known sterilizing machines is that the loose products located in the bags often have to be transferred to storage stations, located between the machine and the station of use, therefore another harmful pass is required, which can negatively affect the sterilization conditions.

Obviously, the storage station, as well as being another element in the sterilization line which could negatively affect the sterilization conditions, negatively affects the complexity, costs, overall bulk and general efficiency of the sterilization line.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

There is therefore a need to perfect a sterilizing machine for loose products which can overcome at least one of the disadvantages of the state of the art.

One purpose of the present invention is therefore to provide a sterilizing machine which guarantees, also during the step of unloading and transfer to another station of use for loose products, optimal and uniform conditions of sterilization of the loose products, thus without interrupting the conditions of sterility obtained in the treatment chamber of the sterilizing machine.

Another purpose of the present invention is to provide a sterilizing machine which allows the direct transfer of the loose products to a possible station of use in an efficient manner and without requiring the use of intermediate storage stations.

Another purpose of the present invention is to provide a sterilizing machine in which the sterilization and unloading cycle is completely automated and limits the intervention of external operators to a minimum.

Another purpose of the present invention is to perfect an efficient and uniform sterilization process, which always guarantees optimal conditions and uniformity of the sterilization.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purpose, a sterilizing machine for loose products in accordance with the present invention, comprises a treatment chamber able to house the loose products to be treated. In some embodiments, the sterilizing machine comprises, downstream of the treatment chamber, a container to transfer the loose products provided with a loading side of the loose products coming from the treatment chamber, an unloading side of the loose products; the container is made to rotate by means of a drive motor around an axis that passes through the loading side and the unloading side; at least one helicoidal guide with several spirals positioned inside the container and configured to move and guide the loose products from the loading side to the unloading side; the container is connected, on the loading side and by means of first connecting and channeling means to the treatment chamber, and is connectable, during use, on the unloading side and by means of second connecting and channeling means, to a station to use the loose products; the first and the second connecting and channeling means are configured to guarantee continuity in the sterilization conditions obtained in the treatment chamber of the sterilization machine.

The container can be a rotatable drum provided with a cylindrical casing comprising an internal chamber where the helicoidal guide develops along the axis of rotation.

The container can comprise on the loading side, a first tubular element communicating with a hopper to receive the loose products connected to an unloading aperture of the treatment chamber and, on the unloading side, a second tubular element to transfer, during use, the loose products toward the station of use.

The container can comprise truncated cone parts to connect with the tubular elements.

The container can have an inclination from the top downward from the loading side to the unloading side.

The first tubular element can be integrated with the rotating container and the hopper comprises another tubular element connected to the first tubular element by means of a rotatable joint.

The sterilizing machine can comprise at least a first valve to open and close the passage of the loose products from the treatment chamber to the loading side of the container.

The rotatable joint can be positioned between the first valve and the first tubular element.

The second tubular element can be integrated with the rotating container and is connected, by means of a rotatable joint, to another tubular element.

The machine can comprise at least a second valve to open and close the passage of the loose products from the container to the station of use.

The rotatable joint of the second tubular element with the other tubular element is positioned between the second tubular element and the second valve.

The invention also concerns a method for sterilizing loose products, carried out in a sterilizing machine for loose products. According to one aspect of the invention, the method comprises loading the loose products to be treated into a treatment chamber; at least a first treatment cycle of the loose products in the treatment chamber; the direct unloading of the loose products to a transfer container located downstream of the treatment chamber, suitable to move the loose products disposed inside it and connectable to a station to use the loose products located downstream of the container; wherein the container is made to rotate, by means of a drive motor around an axis that passes through the loading side and the unloading side, and comprises at least a helicoidal guide with several spirals positioned inside the container and configured to move and guide the loose products from the loading side to the unloading side.

Preferably, during the first treatment cycle a sterilization is also carried out of the transfer container located downstream of the treatment chamber, thanks to the opening of a first valve located downstream of the treatment chamber and upstream of the loading side of the transfer container, the first valve is closed when a treatment cycle subsequent to the first treatment cycle is concluded in the treatment chamber and until said subsequent treatment cycle is completed, so as to keep said transfer container sterilized.

The invention also concerns a line for sterilizing loose products, comprising a sterilizing machine for loose products and a station to use the loose products located downstream of the sterilizing machine.

These and other aspects, characteristics and advantages of the present disclosure will be better understood with reference to the following description, drawings and attached claims. The drawings, which are integrated and form part of the present description, show some forms of embodiment of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some embodiments, given as a non-restrictive example with reference to the attached drawings wherein:

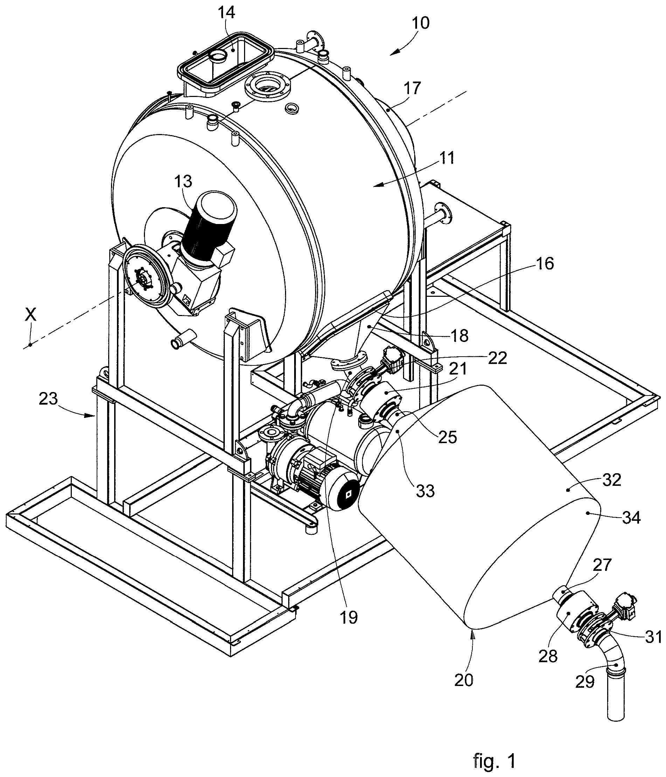

- fig. 1 is a axonometric view of a sterilizing machine according to the present invention;

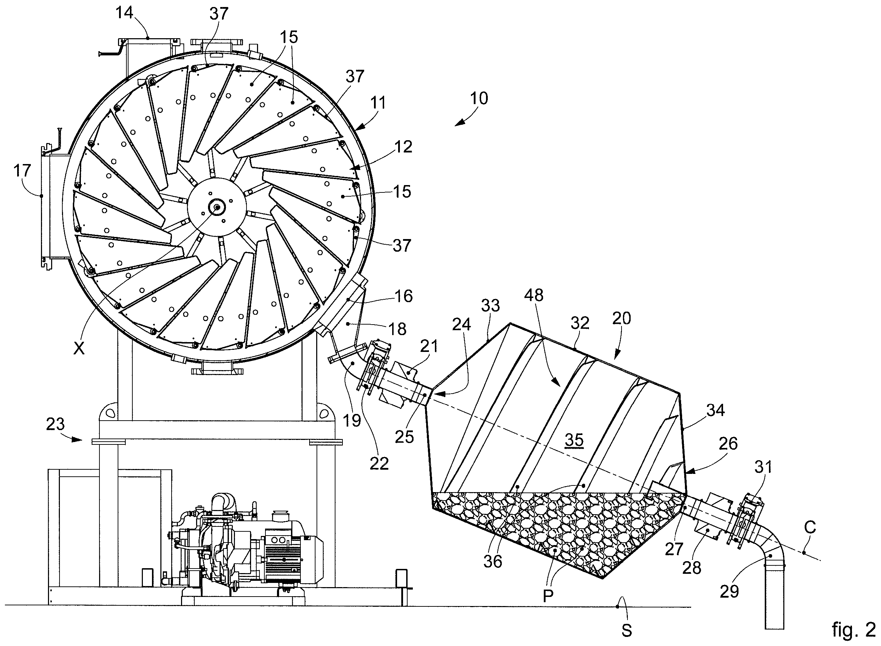

- fig. 2 is a view of the sterilizing machine in lateral elevation and longitudinal section;
- fig. 3 is another lateral view of the sterilizing machine with downstream a station to use the loose products, for example a filling machine.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We will now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

With reference to the attached drawings, a sterilizing machine 10 according to the invention comprises a treatment chamber 11 in which the loose products P to be treated are housed.

By way of non-restrictive example, reference will be made in the following description to a sterilizing machine 10 in whose treatment chamber 11 a rotatable drum 12 is housed, however, the description could refer to a different type of sterilizing machine 10 comprising a treatment chamber 11 for the loose products P without a rotatable drum 12, but provided, for example, with other containing elements of the loose products P to be treated.

The drum 12 can rotate around an axis X and is made to rotate by suitable drive means 13.

The drum 12 is substantially cylindrical in shape and is subdivided into a series of containing sectors 15, able to contain a determinate quantity of loose products P to be treated.

The containing sectors 15 typically have a pyramidal and widened shape proceeding from the center to the periphery of the drum 12.

In proximity to the periphery, each of the containing sectors 15 comprises a door 37, which can be selectively closed or opened to allow the loading of the loose products P to be treated inside the corresponding containing sector 15 and the unloading of the treated loose products P from the containing sector 15.

The treatment chamber 11 comprises a loading aperture 14 of the loose products P to be treated inside each of the containing sectors 15.

In general, the loading aperture 14, as shown, is positioned on an upper part of the treatment chamber 11.

The treatment chamber 11 also comprises a door 17 for possible access to the drum 12 and to the corresponding containing sectors 15.

The treatment chamber 11 also comprises an unloading aperture 16, from which, on each occasion, the loose products P treated in each containing sector 15 exit.

The unloading aperture 16 is preferably positioned in a lower part of the sterilizing machine 10, so that the treated loose products P coming from a determinate containing sector 15 pass through the aperture 16 due to gravity.

Downstream of the unloading aperture 16 a hopper 18 can be provided, to transfer, or unload, the loose products P to a tubular element 19, which can be connected to a container 20 to transfer and unload the treated loose products P, in particular an airtight transfer container 20 comprising a tubular casing 32.

The hopper 18 and tubular element 19 have a mainly downward inclination, so that the loose products P can be transferred by gravity to a loading side 24 of the loose products P in the container 20.

In general, thanks to a support framework 23, the treatment chamber 11 is positioned at a height greater than the container 20, with respect to the ground or support surface S of the sterilizing machine 10.

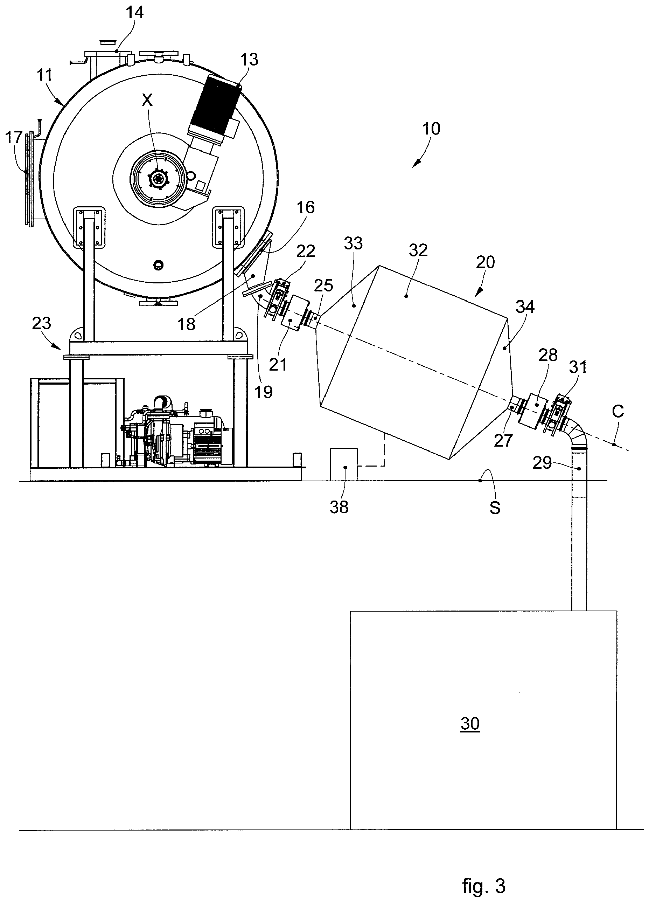

The container 20 is made to rotate around an axis C, which passes through the loading side 24 and unloading side 26, by means of a drive motor 38 (see, for example, fig. 3), so as to keep the loose products P continuously in motion. The axis C can be the central longitudinal axis of the container 20.

Inside it, the container 20 also comprises a helicoidal guide 48 with several spirals 36. The helicoidal guide 48 has the function of guiding the loose products P and moving them uniformly from the loading side 24 to the unloading side 26, during the rotation of the container 20 around the axis C.

The container 20 can be in the form of a rotatable drum provided with a cylindrical casing 32 comprising an internal chamber 35 where the helicoidal guide 48 develops along the axis of rotation C.

The drive motor 38 could be connected in a known manner and by suitable transmission elements to at least one of a pair of tubular elements 25 and 27 protruding respectively from the loading side 24 and from an unloading side 26 of the container 20.

Alternatively, if the container 20 comprises a casing 32 with a cylindrical shape, as shown by way of example in the case here, the drive motor 38 could be connected to a toothed element or suchlike provided around the casing 32.

The container 20 will be supported by a suitable support frame, not shown in the drawings and able to confer upon it, in particular, the inclination shown.

The support frame must provide bearings, rollers or suchlike to support the container 20.

The tubular element 19 can be connected to the tubular element 25 protruding from the loading side 24 of the container 20 by suitable connection means which allow it to rotate around the axis C. The connection means can be, for example, a rotatable joint 21, which also guarantees an airtight seal and allows to maintain the conditions of sterility of the loose products P that exit from the treatment chamber 11. In particular, the tubular element 25 is integral with the container 20 and is therefore made to rotate with the container 20, while the tubular element 19 is stationary.

Between the rotatable joint 21 and the unloading aperture 16, with the possible hopper 18, a first valve 22 is positioned, which opens or closes the passage of loose products P from the treatment chamber 11 of the sterilizing machine 10 to the container 20.

The rotatable joint 21, in particular, also guarantees the desired conditions of sterility in the passage of the loose products P from the first valve 22 to the tubular element 25.

The container 20 as described above comprises the aforesaid unloading side 26 of the loose products P, preferably positioned at a lower height with respect to the loading side 24.

Also the container 20, therefore, has an overall inclination from top to bottom proceeding from the loading side 24 to the unloading side 26 of the loose products P.

The container 20 is provided, in correspondence with the unloading side 26, with the tubular element 27 connectable, by means of a rotatable joint 28, or other suitable connection mean, to a tubular element 29.

In particular, the tubular element 27 is integral with the container 20 and is therefore made to rotate with the container 20, while the tubular element 29 is stationary.

The connection means not only allow the possibility of rotating the container 20 around the axis C, but must also guarantee that the conditions of sterility obtained in the treatment chamber 11 are maintained, for example they can be airtight.

The tubular element 29 can be connected, by means of suitable connection means, to a station 30 for using the loose products, for example an insulated and sterile filling machine.

Downstream of the rotatable joint 28 a second valve 31 is provided, to open and close the passage of the treated loose products P toward the tubular element 29.

The rotatable joint 28, in particular, also guarantees the desired conditions of sterility in the passage of the loose products P from the tubular element 27 and the second valve 31.

The casing 32 of the container 20 can be provided, on the loading side 24 and on the unloading side 26, with truncated cone parts 33 and 34 to connect with the tubular elements 25 and 27.

Inside the casing 32 a chamber 35 is made in which the loose products P are poured and comprising guide means 36 of the said loose products P from the loading side 24 to the unloading side 26, during the rotation of the container 20.

The guide means 36 can be, for example, a helix provided with several suitably spaced spirals, a worm screw or suchlike, which allows to guide the loose products P from the loading side 24 to the unloading side 26 of the container 20.

This movement is required so as to allow a uniform passage and transfer of loose products P from the container 20 to the station of use 30 and also to prevent the loose products P from accumulating in zones of the container 20 lower than the outlet tubular element 27 located in the unloading side 26.

The movement of the loose products P is also required in case the latter are, for example, coated in teflon and therefore tend to stick undesirably to one another.

The inclination of the container 20, therefore of its longitudinal axis C, also allows a possible discharge of treatment water from the chamber 35 of the container 20, by means of suitable discharge elements which will preferably be positioned on the part of the casing 32 that, during use, will be closer to the ground S.

The loose products P to be treated are then introduced into each of the containing sectors 15 of the rotatable drum 12, through the loading opening 14.

For example, it can be hypothesized that the drum 12 is rotated so as to present one containing sector 15 at a time in correspondence with the loading opening 14 and that the door 37 is automatically opened, so as to accommodate a predetermined quantity of loose products P. The door 37 will then be closed again and the loose products P will be introduced into a subsequent containing sector 15.

Once the introduction of the loose products P into the containing sectors 15 of the drum 12 has been completed, a first treatment cycle of the loose products P can be started inside the treatment chamber 11.

In the first treatment cycle, it is possible to keep the first valve 22 open, so as to sterilize not only the loose products P but also the containing sectors 15, the drum 12 and in general the inside of the treatment chamber 11, and also the parts of the sterilizing machine 10 located downstream of the first valve 22.

In this first treatment cycle, therefore, it is also possible to sterilize the container 20, hence its internal chamber 35 with corresponding guide means 36.

It can be hypothesized that, in this first cycle, the second valve 31 is also open, so as to sterilize also the zone downstream of the said second valve 31, for example the tubular element 29.

The sterilization during this first cycle can be performed, for example, up to the entrance of the station of use 30, which, in the case of a filling machine, comprises an access element, such as a stopper or other removable closing element.

When the first treatment cycle is finished, that is, when all the loose products P contained in the various containing sectors 15 have been sterilized, the door 37 of the containing sector 15 which is located in correspondence with the aperture 16 is automatically opened, therefore the loose products P of that containing sector 15 are transferred into the chamber 35 of the container 20.

The loose products P are continuously moved and stirred by means of the drive motor 38 of the container 20, which rotates around the axis C.

Simultaneously, the helicoidal guide 48, which rotates together with the container 20, guides and moves the loose products P from the loading side 24 to the unloading side 26 of the container 20.

As we said, as an alternative to what has been shown, it would be possible to provide a stationary container 20 and rotatable guide means 36, so as to move the loose products P from the loading side 24 to the unloading side 26.

As can be understood, all these operations are carried out, advantageously, keeping the conditions of optimal sterilization created in the treatment chamber 11 unchanged.

From the container 20, the loose products P, by means of the second valve 31, can pass to the station of use 30.

It is possible to hypothesize that the transfer of the loose products P from the container 20 to the station of use 30 takes place, for example, when in the container 20 there is a quantity of loose products P at least equal to the quantity contained in at least one containing sector 15 of the drum 12 of the treatment chamber 11, but, naturally, this quantity can be varied according to the production capacity of the sterilizing machine 10 and/or the station of use 30.

Since the elements downstream of the first valve 22 have been sterilized in this first sterilization cycle, before carrying out a new sterilization cycle with loose products P to be loaded into the treatment chamber 11, the first valve 22 is preferably closed, so that, when the containing sectors 15 of the drum 12 are gradually loaded with loose products P to be treated, all the elements which are located downstream of the first valve 22, therefore mainly the container 20, remain sterile.

Once the operations to sterilize the loose products P of this second cycle have been completed, it is possible to open the first valve 22, so as to proceed with their unloading into the container 20, according to the selected methods and the program of use of the sterilizing machine 10.

The treatment cycles following this second cycle will be completed, therefore, as described above for the second cycle, that is, by alternating the opening and closing of the first valve 22 based on the completion or non-completion of the treatment cycle of the loose products P and, advantageously, so as not to interrupt or negatively influence the continuity of the sterilization conditions obtained in the treatment chamber 11.

The container 20 substantially acts as an intermediate transfer and accumulation element between the treatment chamber 11 and the station of use 30, so as to suitably coordinate the production capacity, and hence the treatment capacity, of the treatment chamber 11 with the productivity of the station of use 30.

If, for example, the station of use 30 is a filling machine, this machine, as is known, has a production capacity lower than the production capacity of the treatment chamber 11 provided with a drum 12, therefore it would not be possible to transfer the treated loose products P directly from the treatment chamber 11 to the filling machine, without using the transfer container 20.

As can be seen from the above description, throughout the sterilization line, comprising the sterilizing machine 10 with the transfer container 20 and the final station of use 30, the loose products P are substantially never subjected to external atmosphere, but are always channeled and contained in sterile elements, such as for example the various tubular passage elements 19, 25, 27, 29, the chamber 35 of the container 20 and so on.

By means of the present sterilizing machine 10, therefore, the optimal sterilization or sterile conditions of the loose products P are always maintained, from the beginning to the end of the treatment cycle, also, therefore, during the delicate unloading step from the treatment chamber 11 of the sterilizing machine 10, thanks in particular to the use of the transfer container 20.

Although the above description refers, in particular and by way of example, to a sterilizing machine 10 with a rotatable drum 12, it is obvious that the container 20 to unload and transfer loose products P could also be used for other types of sterilizing machines 10, for example having different functioning principles from sterilizing machines 10 with a rotatable drum 12.

It is also clear that, although the present invention has been described with reference to some specific examples, a person of skill in the art shall certainly be able to achieve many other equivalent forms of sterilizing machine, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. Sterilizing machine for loose products (P), comprising a treatment chamber (11) able to house the loose products (P) to be treated, said sterilizing machine being **characterized in that** it comprises, downstream of said treatment chamber (11):
a container (20) to transfer said loose products (P) provided with a loading side (24) of said loose products (P) coming from said treatment chamber (11), an unloading side (26) of said loose products (P), said container (20) being made to rotate by means of a drive motor (38), around an axis (C) that passes through said loading side (24) and said unloading side (26);
at least one helicoidal guide (48) with several spirals (36) positioned inside said container (20) and configured to move and guide said loose products (P) from said loading side (24) to said unloading side (26);
said container (20) being connected, on said loading side (24) and by means of first connecting and channeling means (19, 21, 25), to said treatment chamber (11) and being connectable, during use, on said unloading side (26) and by means of second connecting and channeling means (27, 28, 29), to a station (30) to use said loose products (P), said first (19, 21, 25) and said second (27, 28, 29) connecting and channeling means being configured to guarantee continuity in the sterilization conditions obtained in said treatment chamber (11).

2. Machine as in claim 1, **characterized in that** said container (20) is a rotatable drum provided with a cylindrical casing (32) comprising an internal chamber (35) where said helicoidal guide (48) develops along said axis of rotation (C).

3. Machine as in claim 1 or 2, **characterized in that** said container (20) comprises on the loading side (24), a first tubular element (25) communicating with a hopper (18) to receive the loose products (P) connected to an unloading aperture (16) of said treatment chamber (11) and, on the unloading side (26), a second tubular element (27) to transfer, during use, the loose products (P) toward said station of use (30).

4. Machine as in claim 3, tubular **characterized in that** said container (20) comprises truncated cone parts (33, 34) to connect with said tubular elements (25, 27).

5. Machine as in any claim hereinbefore, **characterized in that** said container (20) has an inclination from the top downward from said loading side (24) to said unloading side (26).

6. Machine as in claim 3, **characterized in that** said first tubular element (25) is integrated with the rotating container (20) and said hopper (18) comprises another tubular element (19) connected to said first tubular element (25) by means of a rotatable joint (21).

7. Machine as in any claim hereinbefore, **characterized in that** it comprises at least a first valve (22) to open and close the passage of said loose products (P) from said treatment chamber (11) to said loading side (24) of said container (20).

8. Machine as in claims 6 and 7, **characterized in that** said rotatable joint (21) is positioned between said first valve (22) and said first tubular element (25).

9. Machine as in claim 3, **characterized in that** said second tubular element (27) is integrated with said rotating container (20) and is connected by means of a rotatable joint (28) to another tubular element (29).

10. Machine as in any claim hereinbefore, **characterized in that** it comprises at least a second valve (31) to open and close the passage of said loose products (P) from said container (20) to said station of use (30).

11. Machine as in claims 9 and 10, **characterized in that** said rotatable joint (28) is positioned between said second tubular element (27) and said second valve (31).

12. Method for sterilizing loose products, carried out in a sterilizing machine (10) for loose products (P) as in any claim hereinbefore, said method being **characterized in that** it comprises loading the loose products (P) to be treated into a treatment chamber (11); at least a first treatment cycle of said loose products (P) in said treatment chamber (11); the direct unloading of said loose products (P) to a transfer container (20) located downstream of said treatment chamber (11), suitable to move said loose products (P) disposed inside it and connectable to a station (30) to use said loose products (P) located downstream of said container (20), wherein said container (20) is made to rotate, by means of a drive motor (38) around an axis (C) that passes through said loading side (24) and said unloading side (26) and comprises at least a helicoidal guide (48) with several spirals (36) positioned inside said container (20) and configured to guide and move said loose products (P) from said loading side (24) to said unloading side (26).

13. Method as in claim 12, **characterized in that** during said first treatment cycle a sterilization is also carried out of said transfer container (20) located downstream of said treatment chamber (11), thanks to the opening of a first valve (22) located downstream of a loading side (24) of said treatment chamber (11) and upstream of a loading side (24) of said transfer container (20), said first valve (22) being closed when a treatment cycle subsequent to the first treatment cycle is concluded in said treatment chamber (11) and until said subsequent treatment cycle is completed, so as to keep said transfer container (20) sterilized.

14. Line for sterilizing loose products (P), **characterized in that** it comprises a sterilizing machine (10) as in any of the claims from 1 to 11 and a station (30) to use the loose products (P) located downstream of said sterilizing machine (10).

## Patentansprüche

1. Sterilisationsmaschine für lose Produkte (P), welche eine Behandlungskammer (11) aufweist, die imstande ist, die zu behandelnden losen Produkte (P) unterzubringen, wobei die besagte Sterilisationsmaschine **dadurch gekennzeichnet ist, dass** sie stromabwärts von der besagten Behandlungskammer (11) aufweist:
einen Behälter (20), um die besagten losen Produkte (P) zu transferieren, der mit einer Beladungsseite (24) für die besagten losen Produkte (P), die aus der besagten Behandlungskammer (11) kommen, und einer Entladungsseite (26) für die besagten losen Produkte (P) versehen ist, wobei der besagte Behälter (20) hergestellt ist, um mittels eines Antriebsmotors (38) um eine Achse (C) herum, die durch die besagte Beladungsseite (24) und die besagte Entladungsseite (26) hindurch verläuft, zu rotieren;
mindestens eine schraubenförmige Führung (48) mit mehreren Spiralen (36), die innerhalb des besagten Behälters (20) angeordnet ist und die eingerichtet ist, um die besagten losen Produkte (P) von der besagten Beladungsseite (24) zur besagten Entladungsseite (26) zu bewegen und zu führen;
wobei der besagte Behälter (20) auf der besagten Beladungsseite (24) und mittels erster Verbindungs- und Kanalisierungsmittel (19, 21, 25) mit der besagten Behandlungskammer (11) verbunden ist und während der Verwendung auf der besagten Entladungsseite (26) und mittels zweiter Verbindungs- und Kanalisierungsmittel (27, 28, 29) mit einer Station (30), um die besagten losen Produkte (P) zu verwenden, verbindbar ist, wobei die besagten ersten (19, 21, 25) und die besagten zweiten (27, 28, 29) Verbindungs- und Kanalisierungsmittel eingerichtet sind, um eine Kontinuität der in der Behandlungskammer (11) erhaltenen Sterilisationsbedingungen zu gewährleisten.

2. Maschine gemäß Anspruch 1, **dadurch gekennzeichnet, dass** der besagte Behälter (20) eine drehbare Trommel ist, die mit einem zylindrischen Gehäuse (32) versehen ist, welches eine innere Kammer (35) aufweist, in der sich die besagte schraubenförmige Führung (48) entlang der besagten Drehachse (C) entwickelt.

3. Maschine gemäß Anspruch 1 oder 2, **dadurch gekennzeichnet, dass** der besagte Behälter (20) auf der Beladungsseite (24) ein erstes rohrförmiges Element (25), das mit einem Trichter (18), um die losen Produkte (P) aufzunehmen, in Verbindung steht, der mit einer Entladungsöffnung (16) der besagten Behandlungskammer (11) verbunden ist, und auf der Entladungsseite (26) ein zweites rohrförmiges Element (27), um während der Verwendung die losen Produkte (P) zur besagten Verwendungsstation (30) hin zu transferieren, aufweist.

4. Maschine gemäß Anspruch 3, rohrförmig, **dadurch gekennzeichnet, dass** der besagte Behälter (20) Kegelstumpf-Teile (33, 34), um eine Verbindung mit den besagten rohrförmigen Elementen (25, 27) herzustellen, aufweist.

5. Maschine gemäß irgendeinem vorstehenden Anspruch, **dadurch gekennzeichnet, dass** der besagte Behälter (20) eine Neigung von der Oberseite nach unten hin von der besagten Beladungsseite (24) zur besagten Entladungsseite (26) hat.

6. Maschine gemäß Anspruch 3, **dadurch gekennzeichnet, dass** das besagte erste rohrförmige Element (25) mit dem rotierenden Behälter (20) integriert ist und der besagte Trichter (18) ein weiteres rohrförmiges Element (19) aufweist, das mit dem besagten ersten rohrförmigen Element (25) mittels einer drehbaren Verbindung (21) verbunden ist.

7. Maschine gemäß irgendeinem vorstehenden Anspruch, **dadurch gekennzeichnet, dass** sie mindestens ein erstes Ventil (22), um den Durchgang der besagten losen Produkte (P) von der besagten Behandlungskammer (11) zur besagten Beladungsseite (24) des besagten Behälters (20) zu öffnen und zu schließen, aufweist.

8. Maschine gemäß den Ansprüchen 6 und 7, **dadurch gekennzeichnet, dass** das die besagte drehbare Verbindung (21) zwischen dem besagten ersten Ventil (22) und dem besagten ersten rohrförmigen Element (25) angeordnet ist.

9. Maschine gemäß Anspruch 3, **dadurch gekennzeichnet, dass** das besagte zweite rohrförmige Element (27) mit dem besagten rotierenden Behälter (20) integriert ist und mittels einer drehbaren Verbindung (28) mit einem anderen rohrförmigen Element (29) verbunden ist.

10. Maschine gemäß irgendeinem vorstehenden Anspruch, **dadurch gekennzeichnet, dass** sie mindestens ein zweites Ventil (31), um den Durchgang der besagten losen Produkte (P) von dem besagten Behälter (20) zu der besagten Verwendungsstation (30) zu öffnen und zu schließen, aufweist.

11. Maschine gemäß den Ansprüchen 9 und 10, **dadurch gekennzeichnet, dass** die besagte drehbare Verbindung (28) zwischen dem besagten zweiten rohrförmigen Element (27) und dem besagten zweiten Ventil (31) angeordnet ist.

12. Verfahren zum Sterilisieren von losen Produkten, das in einer Sterilisationsmaschine (10) für lose Produkte (P) gemäß irgendeinem vorstehenden Anspruch durchgeführt wird, wobei das besagte Verfahren **dadurch gekennzeichnet ist, dass** es aufweist: Laden der zu behandelnden losen Produkte (P) in eine Behandlungskammer (11); mindestens einen ersten Behandlungszyklus der besagten losen Produkte (P) in der besagten Behandlungskammer (11); das direkte Entladen der besagten losen Produkte (P) in einen stromabwärts von der besagten Behandlungskammer (11) angeordneten Transferbehälter (20), der geeignet ist, um die innerhalb von ihm angeordneten besagten losen Produkte (P) zu bewegen, und der mit einer stromabwärts von dem besagten Behälter (20) angeordneten Station (30), um die besagten losen Produkte (P) zu verwenden, verbindbar ist, wobei der besagte Behälter (20) hergestellt ist, um mittels eines Antriebsmotors (38) um eine Achse (C) herum, die durch die besagte Beladungsseite (24) und die besagte Entladungsseite (26) hindurch verläuft, zu rotieren, und mindestens eine schraubenförmige Führung (48) mit mehreren Spiralen (36) aufweist, die innerhalb des besagen Behälters (20) angeordnet ist und die eingerichtet ist, um die besagten losen Produkte (P) von der besagten Beladungsseite (24) zur besagten Entladungsseite (26) zu führen und zu bewegen.

13. Verfahren gemäß Anspruch 12, **dadurch gekennzeichnet, dass** während des besagten ersten Behandlungszyklus auch eine Sterilisation des besagten Transferbehälters (20), der stromabwärts von der besagten Behandlungskammer (11) angeordnet ist, durchgeführt wird, dank der Öffnung eines ersten Ventils (22), das stromabwärts von einer Beladungsseite (24) der besagten Behandlungskammer (11) und stromaufwärts von einer Beladungsseite (24) des besagten Transferbehälters (20) angeordnet ist, wobei das erste Ventil (22) geschlossen wird, wenn ein auf den ersten Behandlungszyklus folgender Behandlungszyklus in der besagten Behandlungskammer (11) abgeschlossen ist und bis der besagte folgende Behandlungszyklus beendet ist, um den besagten Transferbehälter (20) sterilisiert zu halten.

14. Linie zum Sterilisieren loser Produkte (P), **dadurch gekennzeichnet, dass** sie eine Sterilisationsmaschine (10) gemäß irgendeinem der Ansprüche 1 bis 11 und eine Station (30), um die losen Produkte (P) zu verwenden, die stromabwärts von der besagten Sterilisationsmaschine (10) angeordnet ist, aufweist.

## Revendications

1. Machine de stérilisation de produits en vrac (P), comprenant une chambre de traitement (11) apte à loger les produits en vrac (P) à traiter, ladite machine de stérilisation étant **caractérisée en ce qu'**elle comprend, en aval de ladite chambre de traitement (11) :
un conteneur (20) pour transférer lesdits produits en vrac (P) pourvu d'un côté de chargement (24) desdits produits en vrac (P) provenant de ladite chambre de traitement (11), d'un côté de déchargement (26) desdits produits en vrac (P), ledit conteneur (20) étant amené à tourner au moyen d'un moteur d'entraînement (38) autour d'un axe (C) qui passe à travers ledit côté de chargement (24) et ledit côté de déchargement (26) ;
au moins un guide hélicoïdal (48) avec plusieurs spirales (36) positionnées à l'intérieur dudit conteneur (20) et configurées pour déplacer et guider lesdits produits en vrac (P) dudit côté de chargement (24) vers ledit côté de déchargement (26) ;
ledit conteneur (20) étant connecté, sur ledit côté de chargement (24) et au moyen de premiers moyens de connexion et de canalisation (19, 21, 25), à ladite chambre de traitement (11) et pouvant être connecté, en utilisation, sur ledit côté de déchargement (26) et au moyen de seconds moyens de connexion et de canalisation (27, 28, 29), à un poste (30) pour utiliser lesdits produits en vrac (P), lesdits premiers (19, 21, 25) et lesdits seconds (27, 28, 29) moyens de connexion et de canalisation étant configurés pour garantir une continuité des conditions de stérilisation obtenues dans ladite chambre de traitement (11).

2. Machine selon la revendication 1, **caractérisée en ce que** ledit conteneur (20) est un tambour rotatif muni d'une enveloppe cylindrique (32) comprenant une chambre interne (35) où ledit guide hélicoïdal (48) se développe le long dudit axe de rotation (C).

3. Machine selon la revendication 1 ou 2, **caractérisée en ce que** ledit conteneur (20) comprend sur le côté de chargement (24) un premier élément tubulaire (25) communiquant avec une trémie (18) pour recevoir les produits en vrac (P) connectée à une ouverture de déchargement (16) de ladite chambre de traitement (11) et, sur le côté de déchargement (26), un second élément tubulaire (27) pour transférer, en utilisation, les produits en vrac (P) vers ledit poste d'utilisation (30).

4. Machine selon la revendication 3, tubulaire **caractérisée en ce que** ledit conteneur (20) comprend des parties tronconiques (33, 34) pour se connecter auxdits éléments tubulaires (25, 27).

5. Machine selon l'une quelconque des revendications précédentes, **caractérisée en ce que** ledit conteneur (20) présente une inclinaison du haut vers le bas depuis ledit côté de chargement (24) vers ledit côté de déchargement (26).

6. Machine selon la revendication 3, **caractérisée en ce que** ledit premier élément tubulaire (25) est intégré au conteneur rotatif (20) et ladite trémie (18) comprend un autre élément tubulaire (19) relié audit premier élément tubulaire (25) au moyen d'un joint rotatif (21).

7. Machine selon l'une quelconque des revendications précédentes, **caractérisée en ce qu'**elle comprend au moins une première vanne (22) pour ouvrir et fermer le passage desdits produits en vrac (P) de ladite chambre de traitement (11) vers ledit côté de chargement (24) dudit conteneur (20).

8. Machine selon les revendications 6 et 7, **caractérisée en ce que** ledit joint rotatif (21) est positionné entre ladite première vanne (22) et ledit premier élément tubulaire (25).

9. Machine selon la revendication 3, **caractérisée en ce que** ledit second élément tubulaire (27) est intégré audit conteneur rotatif (20) et est relié au moyen d'un joint rotatif (28) à un autre élément tubulaire (29).

10. Machine selon l'une quelconque des revendications précédentes, **caractérisée en ce qu'**elle comprend au moins une seconde vanne (31) pour ouvrir et fermer le passage desdits produits en vrac (P) dudit conteneur (20) vers ledit poste d'utilisation (30).

11. Machine selon les revendications 9 et 10, **caractérisée en ce que** ledit joint rotatif (28) est positionné entre ledit second élément tubulaire (27) et ladite seconde vanne (31).

12. Procédé de stérilisation de produits en vrac, mis en oeuvre dans une machine de stérilisation (10) de produits en vrac (P) selon l'une quelconque des revendications précédentes, ledit procédé étant **caractérisé en ce qu'**il comprend le chargement des produits en vrac (P) à traiter dans une chambre de traitement (11) ; au moins un premier cycle de traitement desdits produits en vrac (P) dans ladite chambre de traitement (11) ; le déchargement direct desdits produits en vrac (P) vers un conteneur de transfert (20) situé en aval de ladite chambre de traitement (11), approprié pour déplacer lesdits produits en vrac (P) disposés à l'intérieur de celui-ci et pouvant être connecté à un poste (30) pour utiliser lesdits produits en vrac (P) situé en aval dudit conteneur (20), dans lequel ledit conteneur (20) est amené à tourner, au moyen d'un moteur d'entraînement (38), autour d'un axe (C) qui traverse ledit côté de chargement (24) et ledit côté de déchargement (26) et comprend au moins un guide hélicoïdal (48) avec plusieurs spirales (36) positionnées à l'intérieur dudit conteneur (20) et configurées pour guider et déplacer lesdits produits en vrac (P) dudit côté de chargement (24) vers ledit côté de déchargement (26).

13. Procédé selon la revendication 12, **caractérisé en ce que** pendant ledit premier cycle de traitement, une stérilisation est également effectuée dudit conteneur de transfert (20) situé en aval de ladite chambre de traitement (11), grâce à l'ouverture d'une première vanne (22) située en aval d'un côté de chargement (24) de ladite chambre de traitement (11) et en amont d'un côté de chargement (24) dudit conteneur de transfert (20), ladite première vanne (22) étant fermée lorsqu'un cycle de traitement suivant le premier cycle de traitement est terminé dans ladite chambre de traitement (11) et jusqu'à ce que ledit cycle de traitement suivant soit terminé, de manière à maintenir ledit conteneur de transfert (20) stérilisé.

14. Ligne pour stériliser des produits en vrac (P), **caractérisée en ce qu'**elle comprend une machine de stérilisation (10) selon l'une quelconque des revendications 1 à 11 et un poste (30) pour utiliser les produits en vrac (P) situé en aval de ladite machine de stérilisation (10).
